# EUROPÄISCHE PATENTSCHRIFT

(11) **EP 2 923 640 B1**
(45) Veröffentlichungstag und Bekanntmachung des Hinweises auf die Patenterteilung: **21.07.2021**
(21) Anmeldenummer: 15151431.2
(22) Anmeldetag: 16.01.2015
(51) Int. Cl.: A61B 5/055

(54) **Medizinische Untersuchungseinrichtung**
Medical inspection device
Dispositif d'analyse médicale

(30) Priorität: 25.03.2014 DE 102014205537; 12.06.2014 DE 102014211269
(43) Veröffentlichungstag der Anmeldung: 30.09.2015
(73) Patentinhaber: Siemens Healthcare GmbH, 91052 Erlangen (DE)
(72) Erfinder: Neuber, Wolfgang, 92690 Pressath (DE); Koerth, Michael, 90562 Heroldsberg (DE); Rauh, Peter, 91289 Schnabelwaid (DE)

(56) Entgegenhaltungen:
- WO-A1-2013/073550
- WO-A1-2013/115848
- DE-A1-102010 005 015
- DE-B3-102009 042 873
- US-A1- 2007 016 003
- US-B2- 7 293 308

## Beschreibung

Die Erfindung betrifft eine medizinische Untersuchungseinrichtung, umfassend ein Bildegebungsgerät, beispielsweise ein Magnetresonanztomograph, und eine mobile Patientenliege, wobei an beiden miteinander zu verbindende mechanische Kopplungseinrichtungen zum mechanischen Koppeln der Patientenliege an das Bildgebungsgerät in einer Endposition vorgesehen sind.

Zur Durchführung einer Magnetresonanzuntersuchung wird häufig der Patient auf einer mobilen Patientenliege an den Magnetresonanztomographen gefahren. Diese Patientenliege umfasst zumeist eine horizontal verschiebbare Patientenaufnahme, mit welcher der Patient, wenn die Patientenliege am Magnetresonanztomographen positioniert ist, in die Bohrung gefahren wird. Um eine exakte Positionierung des Patienten zu ermöglichen, wird die mobile Patientenliege mechanisch mit dem Magnetresonanztomographen gekoppelt, so dass beide eine feste Relativposition zueinander einnehmen. Hierzu sind am Magnetresonanztomographen und der Patientenliege entsprechende mechanische Kopplungseinrichtungen vorgesehen, die miteinander zusammenwirken und die Patientenliege in einer Endposition fixieren. Eine exakte und positionsgenaue reversible Positionierung der Patientenliege ist absolut notwendig, um einen störungsfreien und sicheren Behandlungs- respektive Bildaufnahmebetrieb zu gewährleisten.

Bei bisher bekannten Untersuchungseinrichtungen ist als liegenseitige Kopplungseinrichtung ein oder sind mehrere konische Zapfen vorgesehen, der oder die in entsprechende, die tomographenseitigen Kopplungseinrichtungen bildenden Gegenbuchsen eingreifen und bei weiterem Heranschieben der Liege an den Magnetresonanztomographen die richtige Andockposition finden. Die Bewegung der Patientenliege zur Andockstation am Magnetresonanztomographen erfolgt durch manuelles Heranschieben durch das Bedienpersonal. Die Enddurchmesser des oder der Zapfen sowie der einen oder mehreren Gegenbuchsen sind nahezu gleich groß, so dass damit zwangsläufig die Endposition gefunden wird. In der Endposition erfolgt die Verriegelung der Zapfen in den Buchsen, was mittels entsprechender Verriegelungshaken erfolgt, die das Bedienpersonal durch Drücken eines Fußpedals über ein hydraulisches System betätigt.

Zwar kann hierüber eine sichere mechanische Fixierung erreicht werden, jedoch ist die Kopplung relativ umständlich, insbesondere aufgrund der erforderlichen Interaktion des Bedienpersonals. WO 2013/115848 A1 offenbart eine medizinische Untersuchungseinrichtung mit einem Bildgebungsgerät und einer mobilen Patientenliege, welche einen Verriegelungsmechanismus aufweist, der mit einem Kupplungsmechanismus verbunden wird, um eine Bewegung der Patientenliege über einen motorgesteuerten Riemen in eine am Bildgebungsgerät befindliche Endposition zu ermöglichen.

Der Erfindung liegt damit die Aufgabe zugrunde, eine medizinische Untersuchungseinrichtung anzugeben, die demgegenüber verbessert ist und ein leichteres Andocken von Patientenliegen an Bildgebungsgeräten ermöglicht.

Zur Lösung dieser Aufgabe ist bei einer Untersuchungseinrichtung der eingangs genannten Art erfindungsgemäß vorgesehen, dass die mechanische Kopplungseinrichtung des Bildgebungsgeräts eine über einen steuerbaren Antriebsmotor in eine Verriegelungsposition bewegbare Zugeinrichtung umfasst, die zum automatischen Bewegen der Patientenliege einen an der Patientenliege vorgesehenen Mitnehmer vor Erreichen der Endposition greift und durch Bewegen in die Verriegelungsposition mitnimmt, in der die Patientenliege in der Endposition ist.

Alternativ ist zur Lösung dieser Aufgabe bei einer Untersuchungseinrichtung der eingangs genannten Art erfindungsgemäß vorgesehen, dass die mechanische Kopplungseinrichtung der Patientenliege eine über einen steuerbaren Antriebsmotor in eine Verriegelungsposition bewegbare Zugeinrichtung umfasst, die zum automatischen Bewegen der Patientenliege einen an dem Bildegebungsgerät vorgesehenen Mitnehmer vor Erreichen der Endposition greift und durch Bewegen in die Verriegelungsposition mitnimmt, in der die Patientenliege in der Endposition ist.

Erfindungsgemäß ist tomographenseitig ein steuerbarer Antriebsmotor nebst einer über ihn bewegbaren Zugeinrichtung vorgesehen. Diese Zugeinrichtung ist über den Antriebsmotor zwischen einer Löseposition und einer Verriegelungsposition bewegbar. Wird die Patientenliege herangeschoben, so wird mit Erreichen einer entsprechenden Liegenposition der Antriebsmotor entsprechend angesteuert, so dass die Zugeinrichtung aus der Lösestellung bewegt wird. Während dieser Bewegung greift sie den liegenseitigen Mitnehmer. Mit fortgesetzter Bewegung der Zugeinrichtung in Richtung der Verriegelungsposition wird der Mitnehmer zwangsläufig mitgenommen und damit zwangsläufig die Patientenliege in Richtung des Magnetresonanztomographen bewegt. Mit Erreichen der Verriegelungsposition befindet sich die Patientenliege automatisch in der Endposition, in welcher sie exakt und definiert relativ zum Magnetresonanztomographen positioniert ist.

Das Kopplungssystem der erfindungsgemäßen Untersuchungseinrichtung ist wesentlich einfacher aufgebaut, eine hydraulische Betätigungsvorrichtung, die irgendeine Interaktion des Bedienpersonals erfordert, ist nicht vorgesehen. Darüber hinaus wird durch die vorgesehene motorische Verriegelung die Patientenliege selbst in die definierte Endposition bewegt, so dass lediglich im Rahmen des Andockvorgangs seitens des Bedienpersonals die Patientenliege in eine Grundposition relativ zum Bildegbeungsgerät gebracht wird, in welcher die Zugeinrichtung den Mitnehmer greift. Von da an erfolgt der gesamte Kopplungsvorgang automatisch.

Entsprechend einfach erfolgt nach Durchführung der Bildaufnahme die Entkopplung. Es ist lediglich erforderlich, den Antriebsmotor erneut anzusteuern, so dass er die Zugeinrichtung in die entgegengesetzte Richtung bewegt. Dies führt zum zwangsgeführten Herausschieben der Patientenliege aus der Verriegelungsstellung. Erreicht die Zugeinrichtung die Löseposition, so ist die Patientenliege wieder freigegeben, nachdem der Mitnehmer nicht mehr gegriffen ist. Auch hier ist folglich zum eigentlichen Lösen keine Interaktion des Bedienpersonals erforderlich, da auch hier eine automatische Bewegung der Patientenliege aus der gekoppelten Endposition heraus in eine Löseposition erfolgt.

Nach einer ersten Erfindungsausgestaltung kann die Zugeinrichtung ein linear bewegbares Kulissenführungsbauteil mit einer den Mitnehmer aufnehmenden Mitnehmernut aufweisen. Die Mitnehmernut dient als Zwangsführung für den Mitnehmer, der während der Linearbewegung, initiiert über die den Antriebsmotor, in die Nut eingreift und in dieser geführt wird.

Alternativ zur Verwendung eines linear bewegbaren Kulissenführungsbauteils kann auch ein um eine Drehachse drehbares Kulissenführungsbauteil mit einer den Mitnehmer aufnehmenden Mitnehmernut vorgesehen sein. Bei dieser Erfindungsausgestaltung erfolgt anstelle einer Linearbewegung eine Drehbewegung des Kulissenbauteils. Mit Herausdrehen aus der Lösestellung wird der Mitnehmer zwangsläufig in die Mitnehmernut eingeführt, in welcher er bei Fortsetzung der Drehbewegung bis zur Verriegelungsposition zwangsgeführt mitgenommen wird.

Ist ein linear bewegbares Kulissenbauteil vorgesehen, so ist dieses zweckmäßigerweise an einer Linearführung bewegbar geführt, wobei die Zugeinrichtung einen Spindeltrieb mit einer Gewindespindel und einer auf dieser geführten Mutter, mit der das Kulissenführungsbauteil gekoppelt ist, umfasst, wobei die Gewindespindel über den Antriebsmotor antreibbar ist. Über die Linearführung ist das Kulissenbauteil exakt und hinreichend stabil geführt. Über den Spindeltrieb, dessen Gewindespindel über den Antriebsmotor angetrieben wird, kann das Kulissenführungsbauteil auf einfache Weise zwischen der Löse-und der Verriegelungsstellung bewegt werden. Dabei ist das Kulissenführungsbauteil an der Linearführung bevorzugt längs einer senkrecht zur Bewegungsrichtung der Patientenliege verlaufenden Linearachse geführt. Das heißt, es bewegt sich folglich senkrecht zur Patientenliege. Die Mitnehmernut verläuft unter einem Winkel zur Linearachse, der bevorzugt zwischen 30° - 60° liegt. Durch den gewinkelten Verlauf der Mitnehmernut ist die Zwangsführung des Mitnehmers und damit das Heranziehen der Liege an das Bildegebungsgerät sichergestellt, wenn das Kulissenführungsbauteil senkrecht zur Patientenliegenachse bewegt wird. Je nach dem gegebenen Winkel ergibt sich bei gleicher linearer Verschiebungslänge ein kleinerer oder größerer linearer Bewegungsweg der Patientenliege, gesehen von der Eingriffsposition des Mitnehmers in die Nut bis zum Erreichen der Endposition.

Das alternativ verwendbare drehbare Kulissenführungsbauteil ist gemäß einer Weiterbildung der Erfindung an einem drehgelagerten Achsbolzen angeordnet, wobei der Antriebsmotor den Achsbolzen antreibt. Das Kulissenführungsbauteil, das bevorzugt eine Scheibe ist, wird über den Antriebsmotor zwischen der Löse- und der Verriegelungsstellung verdreht, wobei hierfür beispielsweise eine Verdrehung um 180° erfolgt. Die Mitnehmernut verläuft bei dieser Erfindungsausgestaltung gebogen zum Scheibeninneren hin, so dass sich zwangsläufig eine lineare Zugbewegung ergibt.

Gemäß einer besonders vorteilhaften Weiterbildung ist wenigstens ein Sensor vorgesehen, über den die Position der Patientenliege, insbesondere die Position des Mitnehmers erfassbar ist, wobei der Antriebsmotor in Abhängigkeit des Erfassungsergebnisses des Sensors steuerbar ist. Wird die Patientenliege vom Bedienpersonal in Richtung des Bildegbeungsgeräts geschoben, so nähert sie sich zwangsläufig der Andockstation an das Bildegbungsgerät an, also der dortigen Kopplungseinrichtung. Diese Annäherungsbewegung wird über einen Sensor überwacht. Erfasst nun der Sensor, der beispielsweise die Position des Mitnehmers sensiert, dass sich der Mitnehmer in einer entsprechend definierten Grundposition befindet, so wird ein entsprechendes Signal an eine Steuerungseinrichtung gegeben, die daraufhin hierüber quasi getriggert den Antriebsmotor ansteuert, so dass dieser das Kulissenführungsbauteil entsprechend bewegt. Der Sensor erfasst also, wenn sich der Mitnehmer in der Eingriffsposition, in der er in die Nut eingreifen kann, befindet. Sobald das Kulissenführungsbauteil bewegt wird, wird der Mitnehmer in die Nut eingeführt, woraufhin sich die automatische Zugbewegung einstellt. Der Einzug wird also automatisch gestartet.

An der Patientenliege ist bevorzugt ein den Mitnehmer tragendes Führungsbauteil vorgesehen, das beim Heranfahren an den Magnetresonanztomographen zwischen zwei an der dortigen Kupplungseinrichtung angeordnete Führungsrollen greift, von denen es bis zum Erreichen der Endposition geführt ist. Über dieses Führungsbauteil ist folglich eine beidseitige Führung gegeben, das heißt, dass die Patientenliege während der automatischen Einzugsbewegung seitengeführt ist. Diese Führungsrollen sind bevorzugt jeweils doppelt an beiden Seiten des Einzugskanals respektive der Einzugsebene vorgesehen, so dass das Führungsbauteil an jeder Seite folglich doppelt abgestützt respektive geführt ist. Bevorzugt ist das Führungsbauteil eine konische, randseitig gegebenenfalls mit einer Radiuskontur versehene Führungsplatte.

Um auch eine elektrisch Kopplung der Patientenliege mit dem Bildegebungsgerät durch den automatischen Einzug sicherzustellen, was erforderlich ist, um über eine zentrale Steuerungseinrichtung des Bildgebungsgeräts den Bewegungsweg der Tischplatte, die wie einleitend beschrieben automatisch über einen entsprechenden Antriebsmotor verfahrbar ist, zu steuern, sieht die Erfindung ferner an dem Bildgebungsgerät und der Patientenliege elektrische Kopplungseinrichtungen zum elektrischen Koppeln der Patientenliege an das Bildgebungsgerät in der Endposition vor, die spätestens mit Erreichen der Endposition automatisch miteinander verbunden sind. Die Kopplungseinrichtungen sind dabei z. B. als Stecker-Buchsen-Paar ausgeführt. Wird folglich im Rahmen des automatischen Einzugs die Patientenliege in die Endposition gezogen, so werden dabei automatisch auch der Stecker und die Buchse, elektrisch kontaktierend, zusammengefahren. Dies ist, da die Einzugsbewegung eine reine Linearbewegung ist und in eine definierte Endposition mündet, ohne weiteres möglich, das heißt, dass der Stecker exakt in die Buchse eingeführt wird, ohne dass irgend eine Interaktion seitens des Bedienpersonals erforderlich ist. Dabei können den elektrischen Kopplungseinrichtungen beim Zusammenfahren miteinander zusammenwirkende Zentriervorrichtungen zugeordnet sein. Beispielsweise kann an dem liegenseitigen Stecker eine konusartige, ihn umgebende Außenkontur vorgesehen sein, die in eine entsprechende, die bildgebungsgerätseitige Buchse umgebende konusartige Innenkontur gefahren wird und ähnliches.

Mitunter ist die Patientenliege mit einem eigenen Antriebsmotor versehen, der wenigstens zwei liegenseitige Rollen antreibt, so dass die Liege selbstfahrend ist. Über diesen eigenen Antrieb kann die Liege bis nahe an das Bildgebungsgerät fahren. Um bei einer solchen Liegenausgestaltung nun den automatischen Einzug zuzulassen, ohne dass eine liegenseitige Rolle gesperrt ist, nachdem sie wie beschrieben mit den Antriebsmotor gekoppelt ist, ist es zweckmäßig, wenn ein Sensor an der Liege vorgesehen ist, der eine Annäherung der Patientenliege an das Bildgebungsgerät erfasst, wobei die Rollen in Abhängigkeit der Sensorerfassung leerlaufend geschaltet werden. Das heißt, dass entweder die Rollen vom Antriebsmotor entkoppelt werden oder dass der Antriebsmotor leerlaufend geschaltet wird, so dass die Rollen frei drehen können und die Zugeinrichtung nach Greifen des Mitnehmers die Liege in die Endposition ziehen kann.

Weitere Vorteile, Merkmale und Einzelheiten ergeben sich aus dem im Folgenden beschriebenen Ausführungsbeispiel sowie anhand der Zeichnungen. Die Erfindung selbst wird durch die unabhängigen Vorrichtungansprüche 1 und 2 definiert. Weitere Ausführungsbeispiele werden durch Ansprüche 3-15 definiert. Dabei zeigen:
- Fig. 1: eine Prinzipdarstellung einer erfindungsgemäßen medizinischen Untersuchungseinrichtung,
- Fig. 2: eine Teilansicht der Patientenliege und der tomographenseitigen Andockstation,
- Fig. 3: eine vergrößerte Teilansicht der Patientenliege und der Andockstation mit den mechanischen Kopplungseinrichtungen,
- Fig. 4: eine Teilansicht der Andockstation des Magnetresonanztomographen nebst Darstellung des liegenseitigen Mitnehmers in einer Ausgangsstellung vor dem Beginn der mechanischen Kopplung,
- Fig. 5: die Ansicht aus Fig. 4 nach der mechanischen Kopplung,
- Fig. 6: eine Prinzipdarstellung eines Teils einer Andockstation einer zweiten Ausführungsform in einer Seitenansicht,
- Fig. 7: die Darstellung aus Fig. 6 in einer Aufsicht mit Darstellung des liegenseitigen Mitnehmers in einer Grundstellung vor dem Beginn des Kopplungsvorgangs, und
- Fig. 8: die Darstellung gemäß Fig. 7 am Ende des Kopplungsvorgangs.

Im Folgenden wird beispielhaft anhand eines Magnetresonanztomographs ein Ausführungsbeispiel beschrieben. Ausführungsformen mit anderen Bildgebungsgeräten, beispielsweise Computertomographen der Röntgenbildgebung, sind analog aufgebaut.

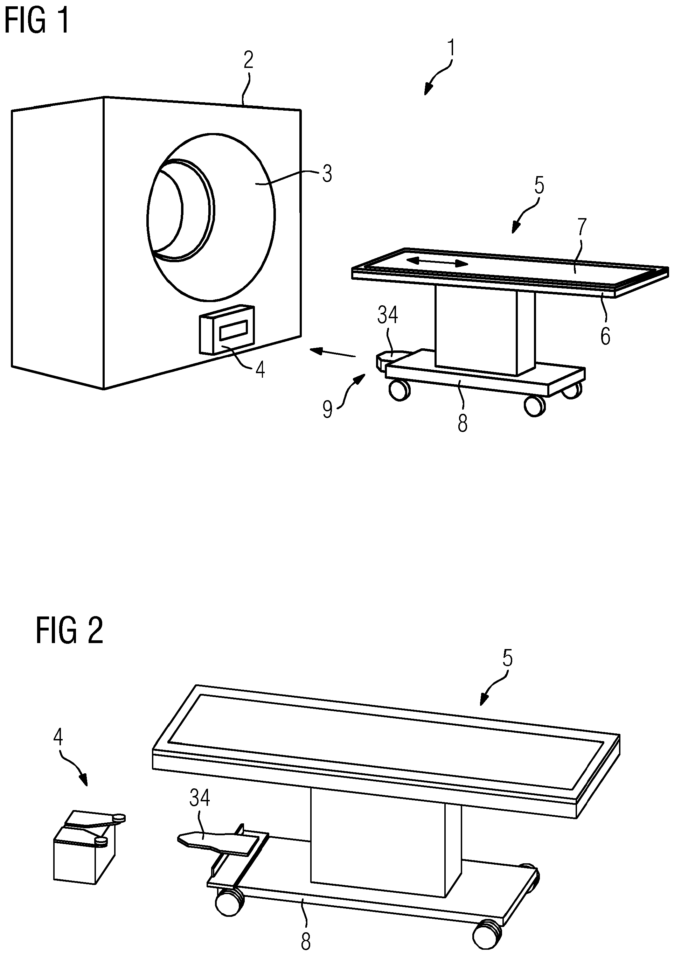

Fig. 1 zeigt eine medizinische Untersuchungseinrichtung 1 in Form einer reinen Prinzipdarstellung, umfassend einen Magnetresonanztomographen 2 mit einer Bohrung 3 sowie einer frontseitig vorgesehenen Andockstation 4, die mechanische Kopplungseinrichtungen, die dem Tomographen zugeordnet sind, umfasst, worauf nachfolgend noch eingegangen wird.

Die Untersuchungseinrichtung 1 umfasst ferner eine Patientenliege 5, die einen Liegentisch 6 mit einer horizontal in Liegenlängsrichtung verfahrbaren Tischplatte 7 umfasst. An einem Fahrgestell 8 ist eine mechanische Kopplungseinrichtung 9 vorgesehen, die mit der Andockstation 4 zusammenwirkt, um die Patientenliege 5 in eine definierte Position relativ zum Magnetresonanztomographen 2 zu bringen. Im Betrieb wird die Patientenliege 5 vom Bedienpersonal entweder manuell oder bei automatischem Fahrbetrieb entsprechend gesteuert in Richtung des Magnetresonanztomographen 2 bewegt, so dass die Andockstation 4 und die Kopplungseinrichtung 9 ineinandergefahren werden, woraufhin ein automatischer Verriegelungsvorgang einsetzt, der nachfolgend näher erläutert wird.

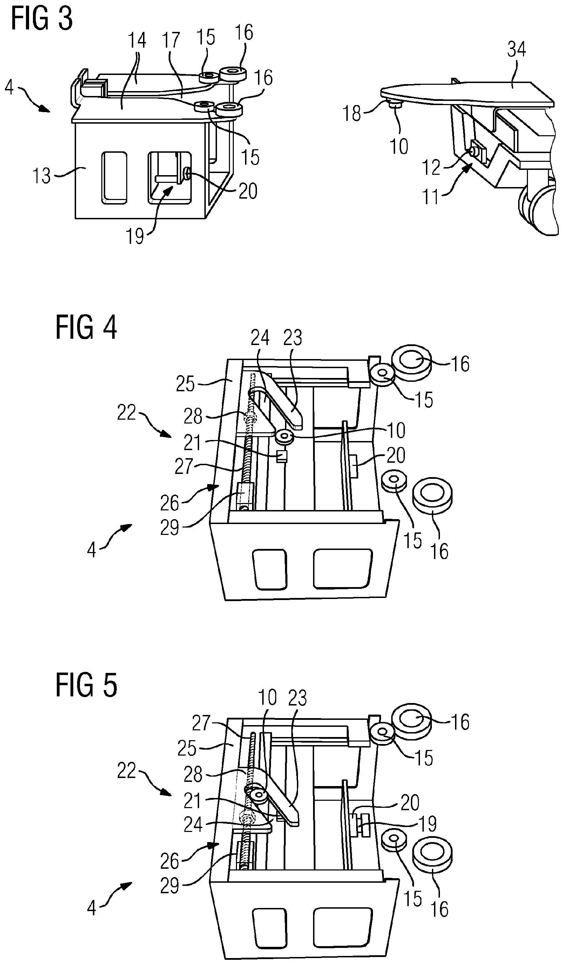

Fig. 2 zeigt in Form einer Detailansicht die Patientenliege 5, sowie als Teil des Magnetresonanztomographen die dortige Andockstation 4. Am Fahrgestell 8 der Patientenliege 5 ist eine Führungsplatte 34 angeordnet, die eine konische Grundform besitzt und randseitig mit einer Radiuskontur versehen ist. An ihrer Unterseite ist ein Mitnehmer 10 angeordnet, der ein zentrales Kopplungsbauteil der Patientenliege 5 darstellt. Des Weiteren ist am Fahrgestell eine elektrische Kopplungseinrichtung 11 umfassend einen Stecker 12 vorgesehen, der mit einer entsprechenden Kopplungseinrichtung an der Andockstation 4 zusammenwirkt, worauf nachfolgend ebenfalls noch eingegangen wird. Dieser Stecker 12 ist exemplarisch in Fig. 3 gezeigt.

Die Andockstation 4 weist ein entsprechendes Gehäuse 13 auf, an dem oberseitig zwei Zentrierplatten 14 vorgesehen sind, an denen am vorderen Ende jeweils zwei Rollenpaare 15, 16 angeordnet sind. Zwischen den beiden Zentrierplatten 14 verbleibt ein sich verjüngender Führungsschlitz 17. Beim Zusammenfahren von Patientenliege 5 und Andockstation 4 wird die Führungsplatte 34 zwischen die Zentrierrollenpaare 15, 16 bewegt und hierüber geführt. Gleichzeitig greift der Mitnehmer 10 respektive ein oberer, im Durchmesser verbreiteter Kragen 18 in den Führungsschlitz 17 und wird in diesem geführt. Die Patientenliege 5 wird soweit verschoben, bis sie in einer Grundposition ist, ausgehend von welcher der automatische mechanische Kopplungsvorgang erfolgen kann. Wie Fig. 3 zu entnehmen ist, ist im Inneren des Gehäuses 13 eine tomographenseitige elektrische Kopplungseinrichtung 19 vorgesehen, die als Buchse 20 ausgeführt ist, in die der Stecker 12 automatisch beim mechanischen Koppeln eingezogen wird, so dass sich gleichzeitig auch eine elektrische Kopplung ergibt.

Fig. 4 zeigt in Form einer Prinzipdarstellung unter Weglassung insbesondere der Zentrierplatten 14, jedoch unter Darstellung der entsprechenden Zentrierrollen 15, 16, die Andockstation 4 sowie als Teil der Patientenliege 5 den Mitnehmer 10. Sämtliche anderen liegenseitigen Teile sind aus Übersichtlichkeitsgründen nicht gezeigt. Wie beschrieben ist die konische Führungsplatte 34 zwischen den Zentrierrollenpaaren 15, 16 seitlich geführt, der Kragen 18 ist im Führungsschlitz 17 geführt. Diese Führung erfolgt bis zum Erreichen einer in Fig. 4 gezeigten Grundstellung, die über einen an der Andockstation 4 verbauten Sensor 21 erfasst wird. In dieser Grundstellung befindet sich der Mitnehmer 10 in einer definierten Position relativ zu der mechanischen Kopplungseinrichtung 22 des Tomographen respektive der Andockstation 4. Diese mechanische Kopplungseinrichtung 22 umfasst ein Kulissenbauteil 23 mit einer Mitnehmernut 24, welches Kulissenführungsbauteil 23 an einer Linearführung 25 in einer Richtung senkrecht zur Bewegungsachse der Patientenliege verschiebbar ist. Hierzu ist ein Spindeltrieb 26 umfassend eine Spindel 27 sowie eine darauf laufende Mutter 28, hier nur gestrichelt dargestellt, vorgesehen. An dieser Mutter 28 ist das Kulissenführungsbauteil befestigt, das wie beschrieben in der Linearführung 25 geführt ist. Über einen Antriebsmotor 29 ist die Gewindespindel 27 drehbar, so dass die Mutter 28 nebst Kulissenführungsbauteil 23 linear bewegt werden kann.

In der in Fig. 4 gezeigten Grundstellung befindet sich der Mitnehmer 10 quasi am Eingang der Mitnehmernut 24, die sich unter einem Winkel von ca. 45° relativ zur Achse der Linearführung 25 erstreckt. Mit Gabe des Erfassungssignals über den Sensor 21 wird über eine nicht näher gezeigte Steuerungseinrichtung des Magnetresonanztomographen der Antriebsmotor 29 angesteuert, so dass die Gewindespindel 27 rotiert und die Mutter 28 nebst Kulissenführungsbauteil 23 längs der Linearführung 25 bewegt wird. Durch diese Bewegung läuft der Mitnehmer 10 zwangsläufig in die Mitnehmernut 24 und in dieser in einer Richtung senkrecht zur Achse der Linearführung 25. Die Endposition ist in Fig. 5 dargestellt. Ersichtlich befindet sich der Mitnehmer 10 am Grund respektive Ende der Mitnehmernut 24 und, ausgehend von der Grundposition gemäß Fig. 4, in einer Einzugsendstellung nahe der Linearführung 25. Das heißt, dass er durch die reine, über den Antriebsmotor 29 automatisierte Linearverschiebung des Kulissenführungsbauteils 23 aktiv in eine Endposition eingezogen wurde. In dieser ist die Patientenliege 5 mechanisch fest verriegelt, nachdem einerseits der Mitnehmer 10 in einer festen Position ist, andererseits die Führungsplatte 34 zwischen dem Zentrierrollenpaaren 15, 16 und über den Kragen 18 in dem Führungsspalt 17 seitlich festgelegt ist. Das Kulissenführungsbauteil stellt folglich eine über den Antriebsmotor 29 nebst Spindeltrieb 26 bewegbare Zugeinrichtung dar, über die die Patientenliege automatisch in eine definierte Endstellung gezogen wird.

Gleichzeitig mit dem automatischen Einziehen kommt es auch zu einer Kopplung des Steckers 19 mit der Buchse 20, so dass die Patientenliege auch elektrisch mit der Andockstation gekoppelt ist und der Betrieb der Patientenliege 5 respektive der automatisierte Verschiebebetrieb der Tischplatte 7 über die Steuerungseinrichtung des Magnetresonanztomographen 2 gesteuert werden kann.

Ähnlich einfach und automatisiert wie die Einzugsbewegung gestaltet sich auch das erneute Lösen der Kopplung. Hierzu ist es lediglich erforderlich, bei Gabe eines entsprechenden Ansteuersignals den Antriebsmotor 29 in die entgegengesetzte Richtung zu betreiben, so dass die Gewindespindel 27 ebenfalls in die entgegengesetzte Richtung rotiert und die Mutter 28 ausgehend von der in Fig. 5 gezeigten Endposition wieder in die in Fig. 4 gezeigte Grundstellung zurückwandert. Hierbei läuft der Mitnehmer 10 aus der Mitnehmernut 24 heraus, er und mit ihm die Patientenliege 5 wird in die entgegengesetzte Richtung automatisch ausgeschoben. Mit Erreichen der Grundstellung gemäß Fig. 4 kann die Patientenliege 5 wieder manuell oder durch ihren eigenen Antrieb vom Tomographen 2 weggefahren werden. Gleichzeitig geht damit natürlich auch eine automatische Entkopplung der elektrischen Kopplung einher.

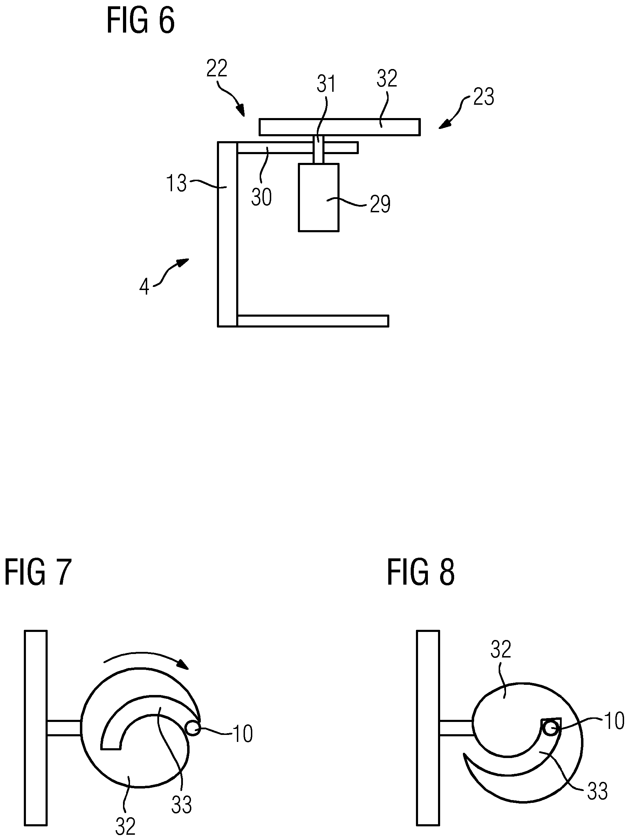

Die Figuren 6 - 8 zeigen in Form einer reinen Prinzipdarstellung eine weitere Ausführungsform einer Andockstation 4 mit deren mechanischen Kopplungseinrichtung 22, wobei soweit möglich für gleiche Bauteile gleiche Bezugszeichen verwendet werden. Am hier nur prinzipiell dargestellten Gehäuse 13 ist an einem geeigneten Träger 30 über einen Achsbolzen 31 ein Kulissenführungsbauteil 23 in Form einer Scheibe 32 positionsfest drehgelagert. Am Achsbolzen 31 setzt der Antriebsmotor 29 an, das heißt, dass der Achsbolzen 31 und mit ihm die Scheibe 32 über den Antriebsmotor 29 gedreht werden können.

Fig. 7 zeigt in Form einer Prinzipdarstellung die Grundstellung, vergleichbar mit der Darstellung gemäß Fig. 4. Der hier dargestellte Mitnehmer 10 wurde wieder über einen nicht näher gezeigten Sensor erfasst, so dass sichergestellt ist, dass er sich in der Grundstellung befindet. Hieraufhin wird über die tomographenseitige Steuerungseinrichtung der Antriebsmotor 29 angesteuert, so dass die Scheibe 32 verdreht wird. Die Scheibe 32 weist wiederum eine Mitnehmernut 33 auf, die sich hier jedoch von außen nach innen windet. In der Grundstellung befindet sich der Mitnehmer 10 am Eingang der Mitnehmernut 33. Rotiert nun die Scheibe, so wird der Mitnehmer 10 in die Mitnehmernut 33 eingezogen, er wandert in ihr bedingt durch die Scheibenrotation zum Nutende, das sich deutlich weiter innen bezogen auf den Scheibenrand befindet. In der Endstellung befindet er sich vollständig in der Mitnehmernut 33 eingezogen, siehe Fig. 8 Ersichtlich bewegt er sich also auch hier linear zum Magnetresonanztomographen 2 hin, was zwangsläufig auch zu einem entsprechenden Einzug der Patientenliege 5 führt. Die Scheibe 32 ist auch hier eine über den Antriebsmotor 29 bewegbare Zugeinrichtung, die die Patientenliege in eine definierte Endstellung automatisch zieht.

Wenngleich hier nicht näher dargestellt, sind selbstverständlich auch die entsprechenden Seitenführungen über die Zentrierplatten 14 mit den Zentrierrollenpaaren 15, 16 an der Andockstation 4 sowie die entsprechende Führungsplatte 34 mit ihrer konischen und gewölbten Seitenform vorgesehen, so dass auch eine entsprechende Seitenführung gegeben ist. Wiederum ist eine exakte, automatisch über den Einzug realisierte mechanische Kopplung gegeben. Gleichzeitig ergibt sich natürlich auch die entsprechende elektrische Kopplung, da auch bei dieser Ausgestaltung der liegenseitige Stecker und die tomographenseitige Buchse vorgesehen sind.

Zum erneuten Lösen der Kopplung wird die Scheibe 32 über den Antriebsmotor 29 in die entgegengesetzte Richtung gedreht. Es kommt wiederum zu einer entsprechenden Zwangsführung des Mitnehmers 10 in der Mitnehmernut 33, er läuft in dieser zum Nutausgang und wird dabei zwangsläufig vom Tomographen weggeschoben, damit einhergehend natürlich auch die Patientenliege 5.

Obwohl die Erfindung im Detail durch das bevorzugte Ausführungsbeispiel näher illustriert und beschrieben wurde, so ist die Erfindung nicht durch die offenbarten Beispiele eingeschränkt und die Erfindung wird durch die Ansprüche definiert. Insbesondere ist auch eine umgekehrte Anordnung möglich, bei der der steuerbare Antriebsmotor 29 in der Patientenliege 5 und der Mitnehmer 10 an einem Bildgebungsgerät angeordnet ist.

### Bezugszeichenliste

- 1: Untersuchungseinrichtung
- 2: Bildgebungsgerät / Magnetresonanztomograph
- 3: Bohrung
- 4: Andockstation
- 5: Patientenliege
- 6: Liegentisch
- 7: Tischplatte
- 8: Fahrgestell
- 9: Kopplungseinrichtung
- 10: Mitnehmer
- 11: Kopplungseinrichtung
- 12: Stecker
- 13: Gehäuse
- 14: Zentrierplatten
- 15: Rollenpaar
- 16: Rollenpaar
- 17: Führungsschlitz
- 18: Kragen
- 19: Kopplungseinrichtung
- 20: Buchse
- 21: Sensor
- 22: Kopplungseinrichtung
- 23: Kulissenbauteil
- 24: Mitnehmernut
- 25: Linearführung
- 26: Spindeltrieb
- 27: Spindel
- 28: Mutter
- 29: Antriebsmotor
- 30: Träger
- 31: Achsbolzen
- 32: Scheibe
- 33: Mitnehmernut
- 34: Führungsplatte

## Patentansprüche

1. Medizinische Untersuchungseinrichtung (1), umfassend ein Bildgebungsgerät (2) und eine mobile Patientenliege, wobei an beiden miteinander zu verbindende mechanische Kopplungseinrichtungen zum mechanischen Koppeln der Patientenliege (5) an das Bildgebungsgerät (2) in einer Endposition vorgesehen sind, wobei die Patientenliege einen Liegentisch (6) mit einer horizontal in Liegenlängsrichtung verfahrbaren Tischplatte (7) und ein Fahrgestell (8) umfasst und die mechanische Kopplungseinrichtung der Patientenliege (5) an dem Fahrgestell (8) angeordnet ist, wobei die mechanische Kopplungseinrichtung (22) des Bildgebungsgeräts (2) eine über einen steuerbaren Antriebsmotor (29) in eine Verriegelungsposition bewegbare Zugeinrichtung (23) umfasst, die zum automatischen Bewegen der Patientenliege (5) einen an der Patientenliege (5) vorgesehenen Mitnehmer (10) vor Erreichen der Endposition greift und durch Bewegen in die Verriegelungsposition mitnimmt, in der die Patientenliege (5) in der Endposition ist **dadurch gekennzeichnet, dass** die Zugeinrichtung ein linear bewegbares oder ein um eine Drehachse drehbares Kulissenführungsbauteil (23) mit einer den Mitnehmer (10) aufnehmenden Mitnehmernut (24, 33) aufweist.

2. Medizinische Untersuchungseinrichtung (1), umfassend ein Bildgebungsgerät (2) und eine mobile Patientenliege, wobei an beiden miteinander zu verbindende mechanische Kopplungseinrichtungen zum mechanischen Koppeln der Patientenliege (5) an das Bildgebungsgerät (2) in einer Endposition vorgesehen sind, wobei die Patientenliege (5) einen Liegentisch (6) mit einer horizontal in Liegenlängsrichtung verfahrbaren Tischplatte (7) und ein Fahrgestell (8) umfasst und die mechanische Kopplungseinrichtung der Patientenliege (5) an dem Fahrgestell (8) angeordnet ist, wobei die mechanische Kopplungseinrichtung (22) der Patientenliege (5) eine über einen steuerbaren Antriebsmotor (29) in eine Verriegelungsposition bewegbare Zugeinrichtung (23) umfasst, die zum automatischen Bewegen der Patientenliege (5) einen an dem Bildgebungsgerät (2) vorgesehenen Mitnehmer (10) vor Erreichen der Endposition greift und durch Bewegen in die Verriegelungsposition mitnimmt, in der die Patientenliege (5) in der Endposition ist, **dadurch gekennzeichnet, dass** die Zugeinrichtung ein linear bewegbares oder ein um eine Drehachse drehbares Kulissenführungsbauteil (23) mit einer den Mitnehmer (10) aufnehmenden Mitnehmernut (24, 33) aufweist.

3. Medizinische Untersuchungseinrichtung nach Anspruch 1 oder 2, **dadurch gekennzeichnet, dass** das linear bewegbare Kulissenführungsbauteil (23) an einer Linearführung (25) bewegbar geführt ist, wobei die Zugeinrichtung einen Spindeltrieb (26) mit einer Gewindespindel (27) und einer auf dieser geführten Mutter (28), mit der das Kulissenführungsbauteil (23) gekoppelt ist, umfasst, wobei die Gewindespindel (27) über den Antriebsmotor (29) antreibbar ist.

4. Medizinische Untersuchungseinrichtung nach Anspruch 3, **dadurch gekennzeichnet, dass** das Kulissenführungsbauteil (23) an der Linearführung (25) längs einer senkrecht zur Bewegungsrichtung der Patientenliege (5) verlaufenden Linearachse geführt ist.

5. Medizinische Untersuchungseinrichtung nach Anspruch 4, **dadurch gekennzeichnet, dass** die Mitnehmernut (24) unter einem Winkel zwischen 30° - 60° zur Linearachse verläuft.

6. Medizinische Untersuchungseinrichtung nach Anspruch nach einem der vorhergehenden Ansprüche, **dadurch gekennzeichnet, dass** das drehbare Kulissenführungsbauteil (23) an einem drehgelagerten Achsbolzen (31) angeordnet ist, wobei der Antriebsmotor (29) den Achszapfen (31) antreibt.

7. Medizinische Untersuchungseinrichtung nach Anspruch 6, **dadurch gekennzeichnet, dass** das Kulissenführungsbauteil (23) eine Scheibe (32) mit einer gebogen zum Scheibeninneren hin geführten Mitnehmernut (33) ist.

8. Medizinische Untersuchungseinrichtung nach einem der vorangehenden Ansprüche, **dadurch gekennzeichnet, dass** wenigstens ein Sensor (21) vorgesehen ist, über den die Position der Patientenliege (5), insbesondere die Position des Mitnehmer (10) erfassbar ist, wobei der Antriebsmotor (29) in Abhängigkeit des Erfassungsergebnisses des Sensors (21) steuerbar ist.

9. Medizinische Untersuchungseinrichtung nach einem der vorangehenden Ansprüche, **dadurch gekennzeichnet, dass** an der Patientenliege (5) oder dem Bildgebungsgerät (2) ein den Mitnehmer (10) tragendes Führungsbauteil (9) vorgesehen ist, das beim Heranfahren an das Bildgebungsgerät (2) zwischen zwei an der dortigen Kopplungseinrichtung (22) angeordnete Führungsrollen (15, 16) greift, von denen es bis zum Erreichen der Endposition geführt ist.

10. Medizinische Untersuchungseinrichtung nach Anspruch 9, **dadurch gekennzeichnet, dass** zwei einander gegenüberliegende Paare von Führungsrollen (15, 16) vorgesehen sind.

11. Medizinische Untersuchungseinrichtung nach Anspruch 9 oder 10, **dadurch gekennzeichnet, dass** das Führungsbauteil eine konische, randseitig gegebenenfalls mit einer Radiuskontur versehene Führungsplatte (34) ist.

12. Medizinische Untersuchungseinrichtung nach einem der vorangehenden Ansprüche, **dadurch gekennzeichnet, dass** an dem Bildgebungsgerät (2) und der Patientenliege (5) ferner elektrische Kopplungseinrichtungen (12, 20) zum elektrischen Koppeln der Patientenliege (5) an das Bildgebungsgerät (2) in der Endposition vorgesehen sind, die spätestens mit Erreichen der Endposition automatisch miteinander verbunden sind.

13. Medizinische Untersuchungseinrichtung nach Anspruch 12, **dadurch gekennzeichnet, dass** die Kopplungseinrichtungen als Stecker-Buchsen-Paar (12, 20) ausgeführt sind.

14. Medizinische Untersuchungseinrichtung nach Anspruch 12 oder 13, **dadurch gekennzeichnet, dass** den elektrischen Kopplungseinrichtungen (12, 20) beim Zusammenfahren miteinander zusammenwirkende Zentriervorrichtungen zugeordnet sind.

15. Medizinische Untersuchungseinrichtung nach einem der vorangehenden Ansprüche, **dadurch gekennzeichnet, dass** die Patientenliege (5) wenigstens zwei über einen liegenseitigen Antriebsmotor antreibbare Rollen aufweist, und dass ein Sensor vorgesehen ist, der eine Annäherung der Patientenliege (5) an das Bildgebungsgerät (2) erfasst, wobei die Rollen in Abhängigkeit der Sensorerfassung leerlaufend geschaltet werden.

## Claims

1. Medical examination facility (1), comprising an imaging apparatus (2) and a mobile patient table, wherein mechanical coupling devices to be connected to each other are provided on both for the purpose of mechanically coupling the patient table (5) to the imaging apparatus (2) in an end position, wherein the patient table comprises a couch (6), with a couch panel (7) that is movable horizontally in the longitudinal direction of the table, and a carriage (8), and the mechanical coupling device of the patient table (5) is arranged on the carriage (8), wherein the mechanical coupling device (22) of the imaging apparatus (2) comprises a pulling device (23), which is movable to a locking position via a controllable drive motor (29) and which, in order to automatically move the patient table (5), engages a driver (10) provided on the patient table (5), before the end position is reached, and carries it by moving to the locking position, in which the patient table (5) is in the end position, **characterized in that** the pulling device has a linearly movable slotted guide component (23), or a slotted guide component (23) rotatable about a rotation axis, with a drive slot (24, 33) receiving the driver (10).

2. Medical examination facility (1), comprising an imaging apparatus (2) and a mobile patient table, wherein mechanical coupling devices to be connected to each other are provided on both for the purpose of mechanically coupling the patient table (5) to the imaging apparatus (2) in an end position, wherein the patient table (5) comprises a couch (6), with a couch panel (7) that is movable horizontally in the longitudinal direction of the table, and a carriage (8), and the mechanical coupling device of the patient table (5) is arranged on the carriage (8), wherein the mechanical coupling device (22) of the patient table (5) comprises a pulling device (23), which is movable to a locking position via a controllable drive motor (29) and which, in order to automatically move the patient table (5), engages a driver (10) provided on the imaging apparatus (2), before the end position is reached, and carries it by moving to the locking position, in which the patient table (5) is in the end position, **characterized in that** the pulling device has a linearly movable slotted guide component (23), or a slotted guide component (23) rotatable about a rotation axis, with a drive slot (24, 33) receiving the driver (10).

3. Medical examination facility according to Claim 1 or 2, **characterized in that** the linearly movable slotted guide component (23) is guided movably on a linear guide (25), wherein the pulling device comprises a spindle drive (26) with a threaded spindle (27) and a nut (28) guided on the latter, to which nut (28) the slotted guide component (23) is coupled, wherein the threaded spindle (27) can be driven via the drive motor (29).

4. Medical examination facility according to Claim 3, **characterized in that** the slotted guide component (23) on the linear guide (25) is guided along a linear axis extending perpendicular to the direction of movement of the patient table (5) .

5. Medical examination facility according to Claim 4, **characterized in that** the drive slot (24) extends at an angle of between 30° and 60° with respect to the linear axis.

6. Medical examination facility according to one of the preceding claims, **characterized in that** the rotatable slotted guide component (23) is arranged on a rotatably mounted axle bolt (31), wherein the drive motor (29) drives the axle bolt (31) .

7. Medical examination facility according to Claim 6, **characterized in that** the slotted guide component (23) is a disc (32) with a curved drive slot (33) leading toward the disc interior.

8. Medical examination facility according to one of the preceding claims, **characterized in that** at least one sensor (21) is provided, via which the position of the patient table (5), in particular the position of the driver (10), can be detected, wherein the drive motor (29) is controllable according to the detection result of the sensor (21).

9. Medical examination facility according to one of the preceding claims, **characterized in that** a guide component (9) supporting the driver (10) is provided on the patient table (5) or on the imaging apparatus (2), which guide component (9), on approaching the imaging apparatus (2), engages between two guide rollers (15, 16) arranged on the coupling device (22) there and is guided by said guide rollers (15, 16) until the end position is reached.

10. Medical examination facility according to Claim 9, **characterized in that** two pairs of guide rollers (15, 16) lying opposite each other are provided.

11. Medical examination facility according to Claim 9 or 10, **characterized in that** the guide component is a conical guide plate (34) which, if appropriate, is provided with a radius contour at the edge.

12. Medical examination facility according to one of the preceding claims, **characterized in that** electrical coupling devices (12, 20) are furthermore provided on the imaging apparatus (2) and on the patient table (5) for the purpose of electrically coupling the patient table (5) to the imaging apparatus (2) in the end position, which coupling devices (12, 20) are automatically connected to each other at the latest when the end position is reached.

13. Medical examination facility according to Claim 12, **characterized in that** the coupling devices are configured as a plug/socket pairing (12, 20).

14. Medical examination facility according to Claim 12 or 13, **characterized in that** the electrical coupling devices (12, 20) are assigned centring devices, which cooperate with each other when brought together.

15. Medical examination facility according to one of the preceding claims, **characterized in that** the patient table (5) has at least two rollers drivable via a table-side drive motor, and **in that** a sensor is provided which detects when the patient table (5) approaches the imaging apparatus (2), wherein the rollers are switched to idle according to the sensor detection.

## Revendications

1. Dispositif (1) d'examen médical, comprenant un appareil (2) d'imagerie et une couchette mobile de patient, dans lequel il est prévu en une position d'extrémité, deux dispositifs d'accouplement mécaniques à relier entre eux pour l'accouplement mécanique de la couchette (5) du patient à l'appareil (2) d'imagerie la couchette du patient comprenant une table (6) de couchette, ayant un plateau (7) de table déplaçable horizontalement dans la direction longitudinale de la couchette, et un chariot (8) et le dispositif d'accouplement mécanique de la couchette (5) du patient est monté sur le chariot (8), dans lequel le dispositif (22) d'accouplement mécanique de l'appareil (2) d'imagerie comprend un dispositif (23) de traction qui peut être mis dans une position de verrouillage par l'intermédiaire d'un moteur (29) d'entraînement pouvant être commandé, qui prend, avant d'atteindre la position finale, pour le déplacement automatique de la couchette (5) du patient un entraîneur (10) prévu sur la couchette (5) du patient et l'entraîne par déplacement dans la position de verrouillage, dans laquelle la couchette (5) du patient est dans la position finale, **caractérisé en ce que** le dispositif de traction a une pièce (23) de guidage à coulisse pouvant être déplacée linéairement ou pouvant tourner autour d'un axe de rotation ayant une rainure (24, 33) d'entraîneur recevant l'entraîneur (10).

2. Dispositif (1) d'examen médical comprenant un appareil (2) d'imagerie et une couchette mobile de patient, dans lequel il est prévu, en une position d'extrémité, deux dispositifs d'accouplement mécaniques à relier entre eux pour l'accouplement mécanique de la couchette (5) du patient à l'appareil (2) d'imagerie la couchette du patient comprenant une table (6) de couchette ayant un plateau (7) de table déplaçable horizontalement dans la direction longitudinale de la couchette et un chariot (8), dans lequel le dispositif (22) d'accouplement mécanique de la couchette (5) du patient comprend un dispositif (23) de traction qui peut être mis dans une position de verrouillage par un moteur (29) d'entraînement pouvant être commandé, qui prend, avant d'atteindre la position finale, pour le déplacement automatique de la couchette (5) du patient, un entraîneur (10) prévu sur l'appareil (2) d'imagerie et l'entraîne par déplacement dans la position de verrouillage, dans laquelle la couchette (5) du patient est dans la position finale, **caractérisé en ce que** le dispositif de traction a une pièce (23) de guidage à coulisse pouvant être déplacée linéairement ou pouvant tourner autour d'un axe de rotation ayant une rainure (24, 33) d'entraîneur recevant l'entraîneur (10).

3. Dispositif d'examen médical suivant la revendication 1 ou 2, **caractérisé en ce que** la pièce (23) de guidage à coulisse pouvant être déplacée linéairement est guidée avec possibilité d'être déplacée sur un guidage (25) linéaire, dans lequel le dispositif de traction comprend une transmission (26) à broche ayant une broche (27) filetée et un écrou (28) qui y est guidé, et auquel la pièce (23) de guidage à coulisse est accouplée, la broche (27) filetée pouvant être entraînée par le moteur (29) d'entraînement.

4. Dispositif d'examen médical suivant la revendication 3, **caractérisé en ce que** la pièce (23) de guidage à coulisse est guidée sur le guidage (25) linéaire le long d'un axe linéaire s'étendant perpendiculairement à la direction de déplacement de la couchette (5) du patient.

5. Dispositif d'examen médical suivant la revendication 4, **caractérisé en ce que** la rainure (24) de l'entraîneur fait un angle compris entre 30° et 60° avec l'axe linéaire.

6. Dispositif d'examen médical suivant l'une des revendications précédentes, **caractérisé en ce que** la pièce (23) tournante de guidage à coulisse est montée sur un tourillon (31) axial monté tournant, le moteur (29) d'entraînement entraînant le tourillon (31) axial.

7. Dispositif d'examen médical suivant la revendication 6, **caractérisé en ce que** la pièce (23) de guidage à coulisse est un disque (32) ayant une rainure (33) d'entraîneur guidée et incurvée vers l'intérieur du disque.

8. Dispositif d'examen médical suivant l'une des revendications précédentes, **caractérisé en ce qu'**il est prévu au moins un capteur (21), par lequel la position de la couchette (5) du patient, notamment la position de l'entraîneur (10), peut être détectée, dans lequel le moteur (29) d'entraînement peut être commandé en fonction du résultat de détection du capteur (21).

9. Dispositif d'examen médical suivant l'une des revendications précédentes, **caractérisé en ce qu'**il est prévu sur la couchette (5) du patient ou sur l'appareil (2) d'imagerie une pièce (9) de guidage portant l'entraîneur (10), qui, lors du passage sur l'appareil (2) d'imagerie, pénètre entre deux galets (15, 16) de guidage montés sur le dispositif (22) d'accouplement qui s'y trouve, par lesquels il est guidé jusqu'à atteindre la position finale.

10. Dispositif d'examen médical suivant la revendication 9, **caractérisé en ce qu'**il est prévu deux paires opposées l'une à l'autre de galets (15, 16) de guidage.

11. Dispositif d'examen médical suivant la revendication 9 ou 10, **caractérisé en ce que** la pièce de guidage est une plaque (34) de guidage conique pourvue du côté du bord le cas échéant d'un contour en rayon.

12. Dispositif d'examen médical suivant l'une des revendications précédentes, **caractérisé en ce qu'**il est prévu sur l'appareil (2) d'imagerie et la couchette (5) du patient en outre des dispositifs (12, 20) de connexion électrique pour la connexion électrique de la couchette (5) du patient à l'appareil (2) d'imagerie dans la position finale, qui sont reliés entre eux automatiquement au plus tard lorsque la position finale est atteinte.

13. Dispositif d'examen médical suivant la revendication 12, **caractérisé en ce que** les dispositifs de connexion sont réalisés sous la forme d'une paire (12, 20) de pièces mâles et de pièces femelles.

14. Dispositif d'examen médical suivant la revendication 12 ou 13, **caractérisé en ce que** les dispositifs (12, 20) de connexion électrique sont associés lors de leur déplacement à des systèmes de centrage coopérant entre eux.

15. Dispositif d'examen médical suivant l'une des revendications précédentes, **caractérisé en ce que** la couchette (5) du patient a au moins deux galets pouvant être entraînés par un moteur d'entraînement du côté de la couchette et **en ce qu'**il est prévu un capteur, qui détecte que la couchette (5) du patient se rapproche de l'appareil (2) d'imagerie, les galets étant montés de manière à tourner à vide en fonction de la détection du capteur.
